# DEMANDE DE BREVET EUROPEEN

(11) **EP 0 761 208 A1**
(43) Date de publication de la demande: **12.03.1997**
(21) Numéro de dépôt: 96401602.6
(22) Date de dépôt: 18.07.1996
(51) Int. Cl.: A61K 9/14

(54) **Procédé de préparation de formes pharmaceutiques sèches et les compositions ainsi réalisées**

(30) Priorité: 27.07.1995 WO PCT/FR95/01009
(71) Demandeur: Laboratoires EFFIK, 91571 Bievres Cédex (FR)
(72) Inventeur: Duclos, Roselyne, FR-76240 Bonsecours (FR); Terracol, Didier, FR-91370 Verrieres le Buisson (FR)
(74) Mandataire: Burtin, Jean-François

(57) **Abrégé**

L'invention se rapporte au domaine de la chimie thérapeutique et, en particulier, à celui de la pharmacotechnie.

Elle a plus particulièrement pour objet des dispersions solides d'agents médicamenteux dans un agent transporteur hydrophile, caractérisées en ce que le principe actif est dissout dans un solvant organique volatil contenant un amide cyclique très hydrophile et en ce que la solution organique est pulvérisée et séchée en un coprécipité qui est broyé. tamisé et dilué avec un excipient ou un véhicule pharmaceutiquement-acceptable.

Le coprécipité sert de matière première pour la réalisation de médicaments.

## Description

La présente invention se rapporte au domaine de la chimie thérapeutique et plus particulièrement à celui de la pharmacotechnie.

L'invention a pour objet un procédé de préparation de formes pharmaceutiques dont la résorption du principe actif par voie digestive est améliorée.

Elle a plus particulièrement pour objet, un procédé de préparation de formes pharmaceutiques sèches destinées à l'administration par la voie digestive dont la résorption est plus rapide ou plus importante.

Elle a spécifiquement pour objet un procédé de préparation de dispersions solides d'agents médicamenteux dans un agent transporteur hydrophile assurant une meilleure solubilité dans les milieux aqueux et une meilleure résorption dans le tractus digestif, procédé dans lequel le ou les principes actifs sont dissous dans un solvant organique contenant un amide cyclique très hydrophile et en ce que la solution organique ainsi obtenue est évaporée à sec puis broyée et tamisée ou éventuellement, utilisée telle quelle.

Lorsque la solution organique amenée à sec est broyée, le produit pulvérulent en résultant peut être dilué avec des excipients ou des véhicules pharmaceutiques ou bien soumis à un enrobage par lit d'air fluidisé.

Les formes pharmaceutiques sèches ainsi réalisées peuvent être conditionnées en comprimés, dragées, pilules, gélules, capsules ou sachets selon les techniques habituelles de la galénique industrielle.

Il est également possible de former, par enrobage en lit d'air fluidisé, un granulé que l'on peut briser, tamiser et comprimer ou bien garder intact et répartir en sachets étanches prêts à l'administration.

On sait que, depuis quelques années, à côté de l'exploration classique des propriétés pharmacologiques et toxicologiques des médicaments, on se soucie de plus en plus de l'aspect quantitatif de la résorption du principe actif. Celle-ci devient de plus en plus l'objet d'études systématiques parce que l'intensité, et parfois la nature de la réponse. sont fonction de la concentration atteinte au niveau du site d'action. L'importance des études pharmacocinétiques et de biodisponibilité a mis en évidence l'intérêt des modifications apportées lors de la préparation des formes galéniques, notamment celles adaptées pour l'administration par voie digestive.

Les médicaments peu solubles dans l'eau ou difficilement salifiables lors du passage dans l'estomac ne sont que partiellement résorbés. La littérature antérieure a montré que la résorption digestive pouvait être modifiée dans un sens favorable par l'étude de la dimension des particules, par l'adjonction d'agents tensioactifs non ioniques ou bien par adjonction d'un agent solubilisant.

La micronisation qui augmente la surface spécifique externe d'un produit pulvérulent constitue déjà une approche du problème, mais ne convient que pour quelques formes pharmaceutiques comme les suspensions ou les gélules. Elle ne peut être une solution générale à ce problème.

L'adjonction d'agents tensioactifs peut augmenter la solubilité du principe actif et par là même, améliorer la cinétique de résorption, mais ne permet pas nécessairement d'obtenir des taux sanguins plus importants. Il est en outre. souvent nécessaire, d'ajouter des quantités très importantes (25 à 50%) d'agent tensioactif pour obtenir un résultat certain. Cette amélioration du passage par voie digestive paraît résulter d'une diminution de la tension superficielle entraînant un accroissement de la perméabilité de la muqueuse digestive.

L'effet bénéfique obtenu par adjonction d'un agent émulsionnant et notamment d'un ester d'acide gras de glucide, découle d'un principe différent. Cet ester augmente la lipophilie de la molécule et rend la barrière intestinale plus aisément franchissable. Néanmoins, ce type de procédé ne donne de résultat qu'avec des molécules très lipophiles et requiert des concentrations élevées en esters d'acide gras et de glucide.

On sait aussi que, pour des substances actives dont l'absorption au niveau du tractus gastro-intestinal est limitée par leur faible solubilité dans les liquides biologiques, une des possibilités offertes aux galénistes pour l'amélioration de leur cinétique de dissolution est la préparation de dispersions solides. Ces dispersions solides (définies par CHIOU W.L et RIEGELMAN S dans J. Pharm Sci. 60, 1281-1302, 1971) constituent des systèmes qui, selon le procédé utilisé pour leur fabrication, peuvent présenter des structures diverses [voir FORD J.L. Pharm. Acta. Helv, 61, 3, 69-88, (1986) BLOCH D.W et SPEISER P.P - Pharm. Acta. Helv. 62, 23-27, (1987)], correspondant à des états cristallographiques différents. L'état vitreux, bien qu'étant un état solide, se rapproche par son désordre structurel de l'état liquide. C'est un état peu ordonné, facile à rompre et qui améliore notablement la vitesse de dissolution des composants les moins solubles.

Néanmoins, malgré le grand nombre de publications relatives à la préparation de dispersions solides, notamment dans les macrogols ou les poloxamères, cette technique n'a pas connu de développement important en raison de son manque de généralité. Dans certains cas, la vitesse de dissolution est grande. Dans d'autres cas, la vitesse de dissolution est plus faible et tend vers une valeur asymptotique. Pour un même médicament et pour une même concentration, on constate des variations très importantes dans les vitesses de solubilité en fonction de la nature de l'agent co-fondant et même dans certains cas, l'impossibilité d'obtenir une solubilisation complète du principe actif, même après un temps de contact prolongé.

Or, il se trouve que la résorption dans le tube digestif se produit pendant un temps court et sur un espace réduit. Il est donc important que dans la formulation selon l'invention, le principe actif soit amené rapidement et complètement sous une forme solubilisée. Le taux d'absorption en dépend.

Il s'est avéré que les meilleures cinétiques d'absorption sont obtenues avec des dispersions dans des polymères très hydrophiles et notamment dans une poly vinylpyrrolidinone sous forme de co-précipité. On obtient un taux de dissolution d'au moins 80% en moins de 10 minutes.

Les études de la Demanderesse ont montré que la vitesse de dissolution était encore augmentée si le solvant organique contenait, en plus, un agent de surface qui améliore davantage la mouillabilité du principe actif et limite éventuellement le phénomène de croissance de cristaux qui s'opère au cours de la conservation des dispersions solides et qui aboutit à une diminution des cinétiques de dissolution en fonction du temps.

Depuis 1961, date à laquelle SEKIGUCHI et OBI ont proposé pour la première lois l'utilisation de dispersions solides, environ 300 publications portant sur le mode de préparation de ces systèmes ainsi que leurs caractéristiques physico-chimiques, galéniques et pharmaceutiques ont paru dans la littérature. Cette technique pharmaceutique, largement décrite, concerne plus d'une centaine de principes actifs dispersés dans au moins un trentaine de transporteurs hydrophiles différents et a fait, par ailleurs, l'objet d'un certain nombre d'articles de synthèse.

Les dispersions solides sont des systèmes dans lesquels un ou plusieurs principes actifs sont dispersés à l'état solide (microparticulaire, voire moléculaire) dans un véhicule inerte solide. Ces dispersions solides sont à distinguer des simples mélanges de poudres désignés communément sous le nom de mélanges physiques.

Deux méthodes sont couramment employées pour la préparation des dispersions solides : la méthode par fusion-solidification qui conduit à la formation de cofondus, et la méthode par dissolution-évaporation qui conduit à la formation de coprécipités. Une méthode mixte, résultant de la combinaison des 2 méthodes précédentes, est parfois citée, mais se trouve rarement employée.

Le procédé selon l'invention utilise la formation de co-précipités par dissolution-évaporation. Le polymère très hydrophile dissout dans le solvant organique est de préférence une polyvinylpyrrolidone de poids moléculaire variant de 10 000 à 50 000, une (N-méthylpyrrolidone), une (N-méthylpipéridinone-2) ou un N-méthyl caprolactames. Ces lactames cycliques choisis sont très solubles dans l'eau et réalisent des co-précipités facilement et complètement solubles dans l'eau.

Le solvant organique utilisé est un solvant qui dissout à la fois le principe actif et le polymère très hydrophile tout en ayant un degré de volatilité suffisamment grand pour pouvoir être éliminé après dissolution de l'ensemble sans avoir recours à des moyens physiques tels que chaleur ou vide, très puissants. De tels solvants sont par exemple des solvants oxygénés comme l'éthanol, l'isopropanol, le tétrahydrofuranne, l'éther isopropylique, l'acétone la méthyl éthyl cétone. le tétrahydropyranne. ou bien des solvants chlorés comme le chlorure de méthylène ou bien encore des mélanges en proportions variables de ces mêmes solvants.

L'agent tensioactif ajouté éventuetllement est de préférence un agent tensioactif non ionique, choisi parmi les esters polyoxyéthyléniques de sorbitanne et d'acide gras saturés ou non saturés, ayant au moins 8 atomes de carbone, les éthers polyoxyéthyléniques d'alcool gras ayant au moins 8 atomes de carbone et, les esters polyoxyéthyléniques d'acide stéarique.

L'agent tensioactif non ionique est choisi parmi ceux qui sont de caractère amphiphile, mais à prédominance hydrophile, d'un HLB > 12, comme par exemple les esters polyoxyéthyléniques de sorbitanne et d'acide gras, comme les Tween 20 à 80, les éthers polyoxyéthyléniques d'alcool gras comme les Brij. 56-58-78-96-97-98-99, G3816 et 3820, G3910 et 3920 ou Ethylan D254 à 257 ou Renex ou Cremophor EL ou le labrafil ou encore des copolymères en bloc, oxyde d'éthyléne/oxyde de propylène comme les Pluronic F68 ou F87 ou le poloxamère 188 ou les synperonics 68 et 108.

L'expérience a montré que les Tweens 40 et 60 et le Cremophor EL ont fourni les meilleurs résultats.

Parmi les principes actifs peu solubles dans l'eau dont l'incorporation dans les formes pharmaceutiques selon l'invention peut être retenue, on citera plus particulièrement :
- des ANTI-INFLAMMATOIRES et ANTALGIQUES
   salsalate
   benorylate
   oxametacine
   piroxicam
   nimesulide
   floctafénine
   ethenzamide
- des IMMUNO-SUPPRESSEURS
   cyclosporine
- des ANTI-HISTAMINIQUES
   terfénadine
   brompheniramine
   chlorpheniramine
- des ANTI-FONGIQUES et ANTI-TRICHOMONAS
   métronidazole
   ornidazole
   dapsone
   itraconazole
- des ANTI-VIRAUX
   cytarabine
- des ANTI-PSYCHOTIQUES
   sulpiride
   sultopride
- des HORMONES
   estradiol et esters
   estrone
   estriol
   progestérone et ses dérivés
- des agents CARDIO-VASCULAIRES
   dobutamine
   diltiazem
   nifédipine et analogues
- des agents ANTI-ULCEREUX
   pirenzépine
- des agents ANTIBACTERIENS
   érythromycine
   fluméquine
   oxytetracycline
   pipéracilline
   céfuroxime
- des agents ANTI-ARYTHMIQUES
   propafénone
   amiodarone
   cordarone
   flécainide
   gallopamil
   verapamil
   dipyridamol
   diisopyramide
- des agents ANTI-MIGRAINEUX
   flunarizine
   dérivés de l'ergot de seigle
- des ANTIDEPRESSEURS
   fluvoxamine
   fluanisone
- des ANTI-HORMONES
   flutamide
- des BRONCHO-DILATATEURS
   tulobuterol
   talinolol
   prénaltérol
- des ANXIOLYTIQUES
   thiothixène
   trazodone
   doxépine
- des VASO-DILATATEURS
   ethaverine
   pentoxyphylline
   eburnamonine
- des DIURETIQUES
   furosémide
   triamtérène
   torasémide
- des agents ANTI-SPASMODIQUES
   flavoxate
   trimébutine
- des agents inhibant l'excrétion du calcium
   clodronate
   pamidronate
- des ANTI-COAGULANTS
   pindione
   tromexan
- et/ou des HYPOCHOLESTEROLEMIANTS, et en particulier, les dérivés de l'acide CHOFIBRIQUE, comme par exemple :
   le clofibrate
   le clofibride
   le fénofibrate
   le gemfibrozil
   le bezafibrate

Le procédé trouve également des applications avec le métronidazole, l'itraconazole, la cyclosporine, la pipéracilline et le céfuroxime.

En principe, la teneur en principe actif dans les compositions selon l'invention est du même ordre de grandeur qu'avec les compositions pharmaceutiques classiques.

Un intérêt tout particulier s'attache aux produits peu ou mal résorbés dans l'intestin comme la progestérone, l'estradiol, l'estrone.

On sait qu'en particulier une substitution en 17α- est nécessaire pour que les agents hormonostéroidiques soient actifs par voie buccale (cyprotérone acétate.

Demegestone, Promegestone, Noréthynodiol diacétate, Médroxyprogestérone, éthynylestradiol), mais cette substitution a pour effet de faire apparaître en même temps, des effets secondaires (action androgène ou antiandrogène, effet estrogène..) qui ne sont pas nécessairement recherchés Il est donc hautement souhaitable d'utiliser les produits hormonaux sous forme native ou sous forme d'un dérivé semblable (Dydrogestérone, 17α-hydroxy progestérone, ester ou éther d'estradiol par exemple) qui ne porte pas ces substituants supplémentaires en 17α-.

On facilite la résorption de telles molécules en les incorporant sous forme de dispersions solides selon l'invention, en présence ou en l'absence d'agents tensioactifs. Dans un tel milieu, le principe actif se dissout aisément et rapidement en présence d'eau.

Dans le cas particulier de dérivés progestéroniques, on peut réaliser des co-précipités renfermant de 10 à 60% en poids de principe actif. La quantité d'agent tensioactif s'échelonne de 0,5 à 20% rapporté à la masse totale de la forme pharmaceutique. Les teneurs préférées s'échelonnent de 15 à 35% en dérivé progestéronique et de 1 à 10% d'agent tensioactif rapporté à la masse totale de la forme pharmaceutique.

On obtient ainsi des co-précipités de dérivés progestéroniques qui se dissolvent pratiquement intégralement dans l'eau ou dans un milieu aqueux en 10 à 20 minutes sous agitation selon la teneur en dérivé progestéronique.

Les essais effectués par la Demanderesse ont montré :
- que la présence d'agent tensioactif comme les polysorbates et les Crémophors améliore, de façon notable, la vitesse de libération du dérivé progestéronique quel que soit le coprécipité. Cette amélioration est d'autant plus importante que la teneur en principe actif augmente dans l'échantillon. C'est ainsi, par exemple, que les taux moyens de dissolution de dérivé progestéronique pour les co-précipités à 30 et 50% poids/poids avec polysorbate, sont respectivement de 95 et 60% en 10 minutes par rapport à 55 et 18% pour ces mêmes échantillons préparés sans polysorbate.
- que la vitesse de dissolution du dérivé progestéronique est indépendante de la teneur en polysorbate 80 dans les coprécipités à 20 et 30% poids/poids de principe actif, car les cinétiques sont pratiquement superposables. En revanche, cette vitesse de dissolution est dépendante du pourcentage pondéral de polysorbate 80 pour les coprécipités à 50% poids/poids. La meilleure cinétique est obtenue avec l'échantillon contenant 10% poids/poids de polysorbate 80 ou de crémophor EL. Ce résultat montre qu'une quantité plus importante d'agent de surface est nécessaire pour faciliter le mouillage et la dissolution de dérivé progestéronique lorsque sa teneur est élevée dans le coprécipité.

Toutefois, au cours de leur conservation, les dispersions solides quoique susceptibles d'évoluer vers des états thermodynamiquement plus stables, par recristallisation ou grossissement de la taille des particules dispersées, les facteurs temps et température ayant un retentissement considérable sur cette transformation -il est apparu nécessaire d'étudier le devenir des coprécipités de progestérone/PVP et progestérone/PVP/polysorbate 80 après 6 mois de conservation à différentes températures.

Les résultats d'essais ont montré que la cinétique de dissolution du dérivé progestéronique à partir de coprécipités conservés 6 mois à 4°C, 20°C et 37°C ne présentent pas de différence significative vis-à-vis de celle établie sur les produits fraîchement préparés, quelle que soit la température de mise en conservation et quelle que soit la concentration en principe actif.

Les coprécipités préparés selon l'invention offrent donc les garanties les plus complètes de stabilité.

Le coprécipité de principe actif se solubilise dans l'eau de manière rapide et totale. Cependant, il s'est avéré ensuite souhaitable d'obtenir une forme galénique administrable par voie orale.

Or, à cause du caractère particulièrement adhérent de la masse, il est très difficile d'obtenir une poudre principe actif+PVP suffisamment fluide, permettant de réaliser des formes galéniques administrables per os.

C'est pourquoi, selon le procédé de l'invention. une fois obtenue, la solution de principe actif+PVP+solvant, il est approprié d'éliminer le solvant en pulvérisant celle solution sur des granulés neutres, dans un courant d'air chaud, afin d'obtenir des granulés enrobés de principe actif, de façon parfaitement homogène.

Les granulés selon l'invention ainsi utilisés sont des granulés neutres de saccharose et de lactose.

Afin de pulvériser cette solution sur les granulés, on utilise un système de lit d'air fluidisé (GLATT) sur lequelle on peut régler la température, le débit d'air et la pression.

Le GLAT sera chargé avec les granulés neutres qui se trouvent donc en suspension dans celui-ci et qui effectuent des mouvements ascendants ou descendants à l'intérieur du flux d'air. Ces mouvements leur permettent d'être enrobés sur toute leur surface par la solution de principe actif au fur et à mesure qu'elle est injectée dans le GLATT.

Afin que chaque particule soit enrobée de façon parfaitement régulière par la solution de principe acitf, l'emploi d'un agent filmogène donnera à cette solution une qualité plastique permettant d'enrober parfaitement chaque granulé. L'agent filmogène utilisé de préférence sera l'hydroxy propylméthyl cellulose (HPMC). L'HPMC étant peu soluble dans l'éthanol, on utilisera de préférence comme solvant, un mélange éthanol/chlorure de méthylène.

Les granulés ainsi enrobés seront ensuite répartis en gélules.

Les exemples suivants sont fournis à titre d'illustration de l'invention sans toutefois la limiter :

### EXEMPLES

Les essais effectués ont donc eu pour objectif l'amélioration des cinétiques de dissolution d'un principe actif, la progestérone. en vue d'une amélioration de la biodisponibilité de cette hormone après administration par voie orale.

Dans une première partie, on a préparé un certain nombre de dispersions solides par la méthode de fusion-solidification et/ou par la méthode de dissolution.

Dans une première partie, on a préparé un certain nombre de dispersions solides par la méthode de fusion-solidification et/ou par la méthode de dissolution-évaporation, en utilisant 5 excipients hydrophiles : le distéarate de saccharose, le polyoxyéthylène glycol 4000, la polyvinylpyrrolidone, l'acide citrique et des phospholipides. Les techniques mises au point ont permis d'obtenir de manière reproductible, des produits présentant une nette amélioration des cinétiques de dissolution de la progestérone L'excipient optimal pour la poursuite de l'étude a été la polyvinylpyrrolidone avec laquelle un coprécipité contenant 20% poids/poids de progestérone présente un taux de dissolution de 80% en 10 minutes.

Dans une seconde partie, on a essayé encore d'optimiser la formulation des coprécipités de progestérone/PVP en modifiant, d'une part la concentration en principe actif dans les échantillons et/ou les conditions d'évaporation du solvant, et en étudiant, d'autre part l'influence d'un agent de surface incorporé dans la formulation à différentes concentrations. On a constaté que le polysorbate 80 ou le crémophor EL améliorait, de façon très significative, la vitesse de dissolution de la progestérone. Les meilleurs résultats sont obtenus avec les coprécipités les plus fortement concentrés.

Dans une troisième partie, on a montré que les cinétiques de dissolution de la progestérone après 6 mois de conservation à 4°C, 20°C et 37°C n'étaient pas significativement modifiées par rapport à celles enregistrées sur les produits fraîchement préparés lorsque les échantillons contenaient du polysorbate 80. Toutefois, en ce qui concerne les échantillons préparés sans polysorbate 80, l'élévation de la température peut être un facteur défavorable à la bonne conservation des échantillons, même si la modification observée a pour effet une amélioration des cinétiques de dissolution de la progestérone. On peut donc supposer que pour des températures proches de 37°C, des modifications des caractéristiques physiques des coprécipités apparaissent, comme par exemple, une transition vitreuse, qui favorise ainsi la libération du principe actif.

Compte tenu de l'ensemble de ces résultats, il apparaît que le coprécipité à 30 à 50% poids/poids de progestérone contenant 5% poids/poids de polysorbate 80 dans la PVP, présente de bonnes caractéristiques galéniques. En effet, les cinétiques de dissolution de l'hormone après conservation sont superposables à celles obtenues sur le produit fraîchement préparé d'une part, et d'autre part fournissent un taux de dissolution supérieur à 95% en moins de 10 minutes.

Un autre avantage du procédé selon l'invention réside dans le fait que les coprécipités renferment une teneur élevée en principe actif et que, de ce fait, on peut réaliser des formulations pharmaceutiques peu volumineuses et, en particulier, des gélules de petit format.

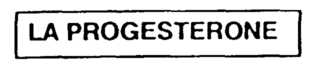

### I - PREPARATION DES DISPERSIONS SOLIDES SELON L'INVENTION

### A - Préparation des mélanges physiques

Des mélanges physiques contenant 20, 30 et 50% poids/poids de progestérone dans la polyvinylpyrrolidone (Kollidon 30 BASF) sont préparés par agitation, pendant 10 minutes au mélangeur TURBULA. Pour chaque échantillon, la quantité totale par lot de fabrication est de 5 grammes.

### B - Préparation des coprécipités de progestérone

Les mélanges physiques contenant des proportions variables de progestérone sont dissous dans une quantité suffisante d'alcool absolu. Les dispersions solides sont obtenues par évaporation du solvant dans les conditions suivantes :
- la vitesse de rotation du ballon est de 100 tours/minute
- la température du bain-marie est portée à 90°C
- la pression à l'intérieur du ballon est amenée progressivement à 200 millibars pour éviter une trop forte ébullition de l'alcool, puis, lorsque la solution présente un aspect visqueux, la pression est à nouveau descendue jusqu'à 50 millibars L'alcool résiduel se trouve ainsi rapidement éliminé et le coprécipité qui présente l'aspect d'une mousse blanche très stable est facilement récupérable en grattant le fond du ballon à l'aide d'une spatule.

Les échantillons ainsi obtenus sont placés 48 heures dans un dessiccateur. avant d'être broyés et tamisés (granulométrie < 100 µm).

### C- Préparation des coprécipités de progestérone-polyvinylpyrrolidone-polysorbate 80

Des mélanges physiques contenant 20, 30 et 50% poids/poids de progestérone ont été préparés avec la polyvinylpyrrolidone, (Kollidon 30 BASF) en remplaçant respectivement 1, 5 ou 10% poids/poids de PVP par une quantité correspondante de polysorbate 80. Le procédé de fabrication de ces coprécipités est identique à celui utilisé pour la préparation des échantillons sans agent de surface, mais le mélange physique est dissout dans une quantité suffisante d'alcool absolu contenant le polysorbate 80 en solution.

Les conditions d'évaporation du solvant sont identiques à celles citées précédemment et les coprécipités présentent également l'aspect d'une mousse blanche friable et facilement récupérable.

**Tableau 1 :**

| préparation des coprécipités 20% poids/poids avec polysorbate 80 | | | | |
|---|---|---|---|---|
| | **Progestérone** | **Polyvinyl pyrrolidone** | **Solution de polysorbate 80 dans alcool (5g/l)** | **Alcool absolu** |
| avec 1% de polysorbate 80 | 1 g | 3,95 g | 10 ml | 80 ml |
| avec 5% de polysorbate 80 | 1 g | 3,75 g | 50 ml | 40 ml |
| avec 10% de polysorbate | 1 g | 3,50 g | 100 ml | 0 |

**Tableau 2 :**

| préparation des coprécipités 30% poids/poids avec polysorbate 80 | | | | |
|---|---|---|---|---|
| | **Progestérone** | **Polyvinyl pyrrolidone** | **Solution de polysorbate 80 dans alcool (5g/l)** | **Alcool absolu** |
| avec 1% de polysorbate 80 | 1,5 g | 3,45 g | 10 ml | 100 ml |
| avec 5% de polysorbate 80 | 1,5 g | 3,25 g | 50 ml | 60 ml |
| avec 10% de polysorbate | 1,5 g | 3,00 g | 100 ml | 10 ml |

**Tableau 3 :**

| préparation des coprécipités 50% poids/poids avec polysorbate 80 | | | | |
|---|---|---|---|---|
| | **Progestérone** | **Polyvinyl pyrrolidone** | **Solution de polysorbate 80 dans alcool (5g/l)** | **Alcool absolu** |
| avec 1% de polysorbate 80 | 2,5 g | 2,45 g | 10 ml | 120 ml |
| avec 5% de polysorbate 80 | 2,5 g | 2,25 g | 50 ml | 70 ml |
| avec 10% de polysorbate | 2,5 g | 2,00 g | 100 ml | 20 ml |

### II - ETUDE DES CINETIQUES DE DISSOLUTION

### A. Technique expérimentale

La chaîne de mesure utilisée pour le contrôle galénique des dispersions solides se compose de 3 éléments :
- un appareil de dissolution DISSOLUTEST DT6 ERWEKA
- une pompe péristaltique 502 SR WATSON-MARLOW
- un spectrophotomètre couplé à un automate de dissolution SAFAS

Chaque essai de dissolution est effectué dans un litre d'eau distillée dégazée à 37°C et de pH voisin de la neutralité.

Une prise d'essai correspondant à 5 mg de progestérone (soit 25 mg de cofondu ou de coprécipité) est introduite dans chacun des six réacteurs au temps To. La vitesse d'agitation des palettes est de 100 tours/mn. Les dosages par absorption dans l'ultra-violet (à 250 nm) sont effectués en continu et en circuit fermé par l'intermédiaire de la pompe péristaltique multicanaux reliant les béchers du Dissolutest aux cuves du spectrophotomètre. Ce dernier, couplé à un enregistreur et à une imprimante, permet de mesurer à intervalles réguliers (toutes les 3 minutes), les variations de la densité optique, elles-mêmes proportionnelles aux variations de concentration du principe actif dans le milieu de dissolution.

### B. Résultats

### B.1 Cinétiques de dissolution des coprécipités de progestérone/PVP

Les cinétiques de dissolution de la progestérone à partir de dispersions solides contenant 20, 30 et 50% poids/poids de principe actif sont représentées au tableau 1.

Les résultats montrent que la vitesse de libération de l'hormone est dépendante de la teneur en principe actif dans le coprécipité. On observe, en effet, un taux de dissolution voisin de 95% en 10 minutes pour le coprécipité à 20% poids/poids contre respectivement 55 et 18% pour les coprécipités à 30 et 50% poids/poids. Ce résultat montre que les meilleurs cinétiques sont obtenues avec les dispersions solides les plus faiblement concentrées, ce qui est probablement en relation avec une plus grande dispersion du principe actif au sein du coprécipité lorsque sa teneur est faible dans l'échantillon.

Par ailleurs, il est intéressant de noter que les cinétiques de dissolution des coprécipités à 20% poids/poids sont plus rapides que celles enregistrées lors de l'étude préliminaire avec les échantillons de même concentration. Ce résultat est dû aux modifications des conditions de préparation, car en diminuant la pression et en augmentant la température du bain-marie, on facilite l'évaporation du solvant et on empêche la cristallisation du principe actif qui reste mal cristallisé ou dans un état plus ou moins amorphe et donc plus soluble.

### B.2 Cinétiques de dissolution des coprécipités progestérone/PVP/Polysorbate 80

Les cinétiques de dissolution de la progestérone à partir des dispersions solides contenant 20, 30 et 50% poids/poids de principe actif avec des proportions variables (1, 5 et 10% poids/poids de polysorbate 80) sont représentés aux tableaux II, III et IV.

**TABLEAU I**

| **Temps (minutes)** | **COPRECIPITES PROGESTERONE-PVP après fabrication** | | |
|---|---|---|---|
| | **20 %** | **30 %** | **50 %** |
| 0 | 0 | 0 | 0 |
| 3 | 77.22 | 33.49 | 5.55 |
| 6 | 88.65 | 45.79 | 9.76 |
| 9 | 92.93 | 52.38 | 17.06 |
| 12 | 95.31 | 57.61 | 18.73 |
| 15 | 96.74 | 62.85 | 22.30 |
| 18 | 97.77 | 65.71 | 23.73 |
| 21 | 98.38 | 68.01 | 27.38 |
| 24 | 99.03 | 71.11 | 30.39 |
| 27 | 99.82 | 74.68 | 33.01 |
| 30 | 99.90 | 77.79 | 35.07 |
| 33 | 99.90 | 80.39 | 37.46 |
| 36 | 99.90 | 83.17 | 39.12 |
| 39 | 99.90 | 85.26 | 41.34 |
| 42 | 99.90 | 87.46 | 43.14 |
| 45 | 99.90 | 88.01 | 44.82 |
| 48 | 99.90 | 89.28 | 46.58 |
| 51 | 99.90 | 91.26 | 48.80 |
| 54 | 99.90 | 92.46 | 50.55 |
| 57 | 99.90 | 94.20 | 51.11 |
| 60 | 99.90 | 94.92 | 52.06 |

**TABLEAU II**

| **Temps (minutes)** | **COPRECIPITES PROGESTERONE-PVP 20% après fabrication avec polysorbate 80** | | |
|---|---|---|---|
| | **1 %** | **5 %** | **10 %** |
| 0 | 0 | 0 | 0 |
| 3 | 87.30 | 86.54 | 80.26 |
| 6 | 94.20 | 94.55 | 92.09 |
| 9 | 97.15 | 98.90 | 97.40 |
| 12 | 99.50 | 99.60 | 100 |
| 15 | 99.80 | 100 | 100 |
| 18 | 100 | 100 | 100 |
| 21 | 100 | 100 | 100 |
| 24 | 100 | 100 | 100 |
| 27 | 100 | 100 | 100 |
| 30 | 100 | 100 | 100 |

**TABLEAU III**

| **Temps (minutes)** | **COPRECIPITES PROGESTERONE-PVP 30% après fabrication avec polysorbate 80** | | |
|---|---|---|---|
| | **1 %** | **5 %** | **10 %** |
| 0 | 0 | 0 | 0 |
| 3 | 79.59 | 83.44 | 78.66 |
| 6 | 88.88 | 93.60 | 91.04 |
| 9 | 92.85 | 96.94 | 96.01 |
| 12 | 96.74 | 98.40 | 98 |
| 15 | 98.23 | 99.40 | 99.70 |
| 18 | 99.86 | 99.70 | 100 |
| 21 | 100 | 100 | 100 |
| 24 | 100 | 100 | 100 |
| 27 | 100 | 100 | 100 |
| 30 | 100 | 100 | 100 |

**TABLEAU IV**

| **Temps (minutes)** | **COPRECIPITES PROGESTERONE-PVP 50% après fabrication avec polysorbate 80** | | |
|---|---|---|---|
| | **20 %** | **30 %** | **50 %** |
| 0 | 0 | 0 | 0 |
| 3 | 22.38 | 36.82 | 44.91 |
| 6 | 37.93 | 51.90 | 60.10 |
| 9 | 46.42 | 61.50 | 69.70 |
| 12 | 54.12 | 67.22 | 74.40 |
| 15 | 58.65 | 71.98 | 78.90 |
| 18 | 62.38 | 76.36 | 83.50 |
| 21 | 65.15 | 80.47 | 89.84 |
| 24 | 68.49 | 81.66 | 93.96 |
| 27 | 70.63 | 83.04 | 95.55 |
| 30 | 72.38 | 84.52 | 96.79 |
| 33 | 74.28 | 86.31 | 98.25 |
| 36 | 75.39 | 87.49 | 99.49 |
| 39 | 77.14 | 88.49 | 100 |
| 42 | 78.65 | 89.12 | 100 |
| 45 | 80.23 | 90.71 | 100 |
| 48 | 81.57 | 91.76 | 100 |
| 51 | 82.38 | 92.58 | 100 |
| 54 | 82.93 | 92.93 | 100 |
| 57 | 83.88 | 93.17 | 100 |
| 60 | 85.15 | 93.92 | 100 |

### III - ETUDE DES CINETIQUES DE DISSOLUTION APRES CONSERVATION

### A. Cinétiques de dissolution des coprécipités de progestérone-PVP

Les cinétiques de dissolution de la progestérone à partir des dispersions solides contenant 20, 30 et 50% poids/poids de principe actif après 6 mois de conservation à 4, 20 et 37°C sont représentées sur les tableaux V, VI et VII.

Comme précédemment, on observe que la vitesse de libération de l'hormone est dépendante de sa teneur dans le coprécipité. Toutefois, la comparaison des cinétiques de dissolution après 6 mois de conservation, par rapport à celles mesurées avec ces mêmes produits fraîchement préparés, montre que l'augmentation de la température de conservation semble améliorer la vitesse de dissolution de la progestérone dans les coprécipités les plus fortement concentrés. Le résultat le plus significatif est obtenu avec le coprécipité à 50% poids/poids conservé à 37°C pour lequel on obtient un taux de dissolution de 95% après une heure contre 52% pour ce même produit fraîchement préparé.

### B. Cinétiques de dissolution des coprécipités de progestérone-PVP-Polysorbate 80

Les cinétiques de dissolution de la progestérone à partir des dispersions solides contenant des proportions variables de polysorbate 80 et après 6 mois de conservation à 4, 20 et 37°C sont représentées sur les tableaux VIII et IX.

Ces résultats montrent que la cinétique de libération de l'hormone à partir des coprécipités conservés 6 mois à 4, 20 et 37°C ne présente pas de différence significative et est pratiquement superposable à celle enregistrée sur les produits fraîchement préparés. La température ne semble donc pas jouer un rôle déterminant dans la conservation des échantillons contenant du polysorbate 80, au cours des 6 premiers mois.

**TABLEAU V**

| **Temps (minutes)** | **COPRECIPITES PROGESTERONE-PVP après conservation 6 mois à 4°C** | | |
|---|---|---|---|
| | **20 %** | **30 %** | **50 %** |
| 0 | 0 | 0 | 0 |
| 3 | 78.87 | 32.22 | 8.01 |
| 6 | 86.80 | 42.77 | 15 |
| 9 | 89.90 | 48.65 | 20.79 |
| 12 | 91 | 52.30 | 25.39 |
| 15 | 92.90 | 56.26 | 29.76 |
| 18 | 94.60 | 58.49 | 33.33 |
| 21 | 96 | 60.71 | 37.14 |
| 24 | 97.80 | 63.17 | 40.24 |
| 27 | 98.80 | 65.28 | 42.93 |
| 30 | 100 | 68.41 | 46.03 |
| 33 | 100 | 70.23 | 47.93 |
| 36 | 100 | 71.82 | 51.11 |
| 39 | 100 | 73.96 | 52.14 |
| 42 | 100 | 75.07 | 54.20 |
| 45 | 100 | 78.88 | 56.35 |
| 48 | 100 | 79.93 | 57.62 |
| 51 | 100 | 80.95 | 59.84 |
| 54 | 100 | 82.06 | 60.95 |
| 57 | 100 | 83.09 | 62.54 |
| 60 | 100 | 83.80 | 64.20 |

**TABLEAU VI**

| **Temps (minutes)** | **COPRECIPITES PROGESTERONE-PVP après conservation 6 mois à température ambiante** | | |
|---|---|---|---|
| | **20 %** | **30 %** | **50 %** |
| 0 | 0 | 0 | 0 |
| 3 | 83.33 | 30.87 | 12.40 |
| 6 | 90.79 | 42.14 | 22.69 |
| 9 | 93.25 | 47.62 | 30.47 |
| 12 | 95.30 | 53.57 | 37.77 |
| 15 | 96.90 | 55.95 | 42.85 |
| 18 | 97.90 | 60.79 | 47.69 |
| 21 | 98.80 | 62.30 | 52.22 |
| 24 | 99.80 | 64.20 | 55.70 |
| 27 | 100 | 67.90 | 58.48 |
| 30 | 100 | 70.48 | 61.98 |
| 33 | 100 | 71.98 | 64.68 |
| 36 | 100 | 75.16 | 66.81 |
| 39 | 100 | 76.90 | 69.67 |
| 42 | 100 | 78.57 | 71.58 |
| 45 | 100 | 80.32 | 72.93 |
| 48 | 100 | 81.83 | 75.63 |
| 51 | 100 | 82.78 | 77.23 |
| 54 | 100 | 83.01 | 78.48 |
| 57 | 100 | 83.65 | 79.99 |
| 60 | 100 | 85.32 | 81.35 |

**TABLEAU VII**

| **Temps (minutes)** | **COPRECIPITES PROGESTERONE-PVP après conservation 6 mois à 37°C** | | |
|---|---|---|---|
| | **20 %** | **30 %** | **50 %** |
| 0 | 0 | 0 | 0 |
| 3 | 82.69 | 43.73 | 18.02 |
| 6 | 90.55 | 65.63 | 32.30 |
| 9 | 96.42 | 71.50 | 42.54 |
| 12 | 97.69 | 74.92 | 51.67 |
| 15 | 98.96 | 77.22 | 58.10 |
| 18 | 99.44 | 78.09 | 64.21 |
| 21 | 100 | 80.01 | 68.33 |
| 24 | 100 | 81.77 | 71.91 |
| 27 | 100 | 83.09 | 76.03 |
| 30 | 100 | 83.96 | 79.13 |
| 33 | 100 | 84.28 | 80.79 |
| 36 | 100 | 85.58 | 83.89 |
| 39 | 100 | 86.22 | 85.72 |
| 42 | 100 | 87.34 | 86.75 |
| 45 | 100 | 88.65 | 88.76 |
| 48 | 100 | 89.20 | 90.86 |
| 51 | 100 | 89.84 | 91.26 |
| 54 | 100 | 90.46 | 92.26 |
| 57 | 100 | 91.19 | 93.56 |
| 60 | 100 | 91.98 | 94.66 |

**TABLEAU VIII**

| **Temps (minutes)** | **COPRECIPITES PROGESTERONE-PVP 20 % avec polysorbate 80 après 6 mois à 4°C** | | |
|---|---|---|---|
| | **1 %** | **5 %** | **10 %** |
| 0 | 0 | 0 | 0 |
| 3 | 78.65 | 84.85 | 69.95 |
| 6 | 90.70 | 93.70 | 87.10 |
| 9 | 93.40 | 96.50 | 93 |
| 12 | 95 | 98.20 | 95.80 |
| 15 | 96.70 | 99.30 | 97.50 |
| 18 | 97.70 | 100 | 98.40 |
| 21 | 98.20 | 100 | 99.30 |
| 24 | 99.20 | 100 | 100 |
| 27 | 99.90 | 100 | 100 |
| 30 | 100 | 100 | 100 |

**TABLEAU IX**

| **Temps (minutes)** | **COPRECIPITES PROGESTERONE-PVP 50% avec polysorbate 80 après 6 mois à 37°C** | | |
|---|---|---|---|
| | **1 %** | **5 %** | **10 %** |
| 0 | 0 | 0 | 0 |
| 3 | 31.78 | 33.41 | 29.10 |
| 6 | 49.18 | 47.39 | 48.29 |
| 9 | 55.12 | 56.66 | 57.80 |
| 12 | 62.27 | 62.35 | 65.36 |
| 15 | 65.20 | 68.12 | 71.13 |
| 18 | 68.53 | 70.64 | 75.12 |
| 21 | 71.05 | 75.36 | 79.34 |
| 24 | 72.52 | 78.12 | 81.13 |
| 27 | 75.12 | 80.23 | 82.50 |
| 30 | 76.42 | 81.86 | 84.14 |
| 33 | 77.31 | 83.57 | 85.77 |
| 36 | 78.29 | 84.46 | 86.01 |
| 39 | 79.75 | 86.74 | 87.43 |
| 42 | 80.73 | 88.53 | 88.86 |
| 45 | 82.27 | 89.42 | 90.08 |
| 48 | 82.60 | 89.67 | 90.80 |
| 51 | 83.08 | 91.29 | 91.38 |
| 54 | 85.04 | 91.70 | 92.35 |
| 57 | 85.12 | 92.35 | 92.84 |
| 60 | 85.60 | 93.81 | 93.41 |

Etude comparative de la progestéronémie plasmatique (mesure par dosage radioimmunologique après extraction de la progestérone par chromatographie) après absorbtion orale à jeun chez huit volontaires saines ménauposées.
(Etude croisée chez des volontaires hospitalisées et à jeun depuis la veille au soir, repas pris après le prélèvement effectué à la quatrième heure suivant l'ingestion du produit).

| *Produit étudie* | *Expression des résultats* | *Cmax (ng/ml)* | *Tmax (h)* | *SSC (ng.ml*^{*-1*}*.h)* | *Rapport de moy. géométrique des SSC vs Utrogestan 200 mg* |
|---|---|---|---|---|---|
| EF 487 2x100 mg | moyenne, | 115,46 | 0,69 | 144,71 | 5,66 |
| | SEM | 26,73 | 0,09 | 34,28 | |
| | min - max | 7,80 231,58 | 0,50 1,00 | 18,62 338,98 | |
| | CV | 0,68 | 0,38 | 0,69 | |
| EF 487 2x70 mg | moyenne, | 67,25 | 0,75 | 73,99 | 3,90 |
| | SEM | 21,20 | 0,09 | 22,46 | |
| | min - max | 6,39 167,62 | 0,50 1,00 | 15,36 209,95 | |
| | CV | 0,91 | 0,36 | 0,90 | |
| EF 487 2x35 mg | moyenne, | 10,82 | 0,69 | 13,39 | 0,56 |
| | SEM | 5,24 | 0,09 | 3,80 | |
| | min - max | 2,52 47,82 | 0,50 1,00 | 5,47 40,07 | |
| | CV | 1,40 | 0,38 | 0,84 | |
| Ultrogestan 2x100 mg | moyenne, | 8,61 | 2,06 | 23,51 | |
| | SEM | 3,51 | 0,58 | 4,91 | |
| | min - max | 0,57 27,14 | 1 6 | 7,13 51,90 | |
| | CV | 1,19 | 0,80 | 0,65 | |

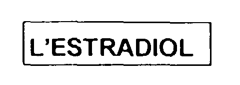

### I - PREPARATION DES DISPERSIONS SOLIDES SELON L'INVENTION

### A - Matières premières

Les matières premières utilisées sont :
- l'hémihydrate d'Estradiol, Art Nr 680025, Charge 24056446, Schering AG, Berlin.
- la polyvinyl pirrolidone (Kollidon 30, Nr 56.0902) (BASF Aktiengesellschaft, 67056 Ludwigshafen, Germany).
- le Tween 80 (polyoxyethylène sorbitan monooleate, AB 397) (ICI Chemicals, Niederlassung der Deutsche ICI Gmbh, Goldschmidtstr 100 D 4300 Essen 1).
- l'alcool ethylique absolu (No CEE 603-002-00-5) (Distillerie Hauguel, 76700 Gonfreville L'Orcher).

### B- Le matériel

Le matériel utilisé pour la préparation des dispersions solides se compose d':
- une balance électronique A 120 Sartorius (Osi, 141, rue de Javel, 75015 Paris),
- un évaporateur rotatif RE 140 Buchi (Roucaire, 20 Av. de l'Europe, 78143 Velezy).

### C- Préparation des dispersions solides par dissolution-évaporation

Les mélanges physiques contenant 10% poids/poids d'estradiol ont la composition suivante:
0,5 g estradiol
4,25 g PVP
0,25 g Tween 80, soit 50 ml d'une solution à 5 g/l dans l'alcool absolu
20 ml d'alcool absolu suffisant pour dissoudre l'estradiol et la PVP.
Ces mélanges sont dissouts dans une quantité suffisante d'alcool absolu (il faut environ 50 ml d'alcool par gramme d'estradiol, 10 ml d'alcool par gramme de PVP).
Les dispersions solides sont obtenues par évaporation du solvant sous vide (P=200 millibars) durant 15 minutes puis la pression est diminuée à son minimum ( environ 40 millibars) pendant 15 minutes. La température du bain-marie est de 60°C et la vitesse de rotation du ballon est de 100 tours / minute.

Les échantillons récupérés sont placés dans un dessicateur pendant 48 heures puis sont broyés et tamisés. Les essais galéniques sont effectués sur des échantillons de granulométrie inférieure à 100 micromètres.

### II - ETUDE DES CINETIQUES DE DISSOLUTION

### A- Technique expérimentale

La chaine de mesure utilisée pour le contrôle galénique des dispersions solides se compose de trois éléments :
- un appareil de dissolution DISSOLUTEST DT6 ERWEKA (Euraf, 55 rue Deschanel, 92400 Courbevoie).
- une pompe péristaltique 502 SR WATSON-MARLOW (Prolabo).
- un spectrophotomètre couplé à un automate de dissolution SAFAS (Prolabo). Chaque essai de dissolution est effectué dans un litre d'eau distillée dégazée à 37 °C et de pH voisin de la neutralité.

Une prise d'essai correspondant à 4 mg d'estradiol (soit 25 mg de coprécipité) est introduite dans chacun des six réacteurs au temps t=O. La vitesse d'agitation des palettes est de 100 tours/min. Les dosages par absobtion dans l'ultra-violet (à 278 nm) sont effectués en continu et en circuit fermé par l'intermédiaire de la pompe péristaltique multicanaux reliant les bêchers du dissolutest aux cuves du spectrophotomètres. Ce dernier, couplé à un enregistreur et à une imprimante, permet de mesurer à intervalles réguliers (toutes les trois minutes) les variations de la densité optique, elles - mêmes proportionnelles aux variations de la concentration du principe actif dans le milieu de dissolution.

### B- Résultats

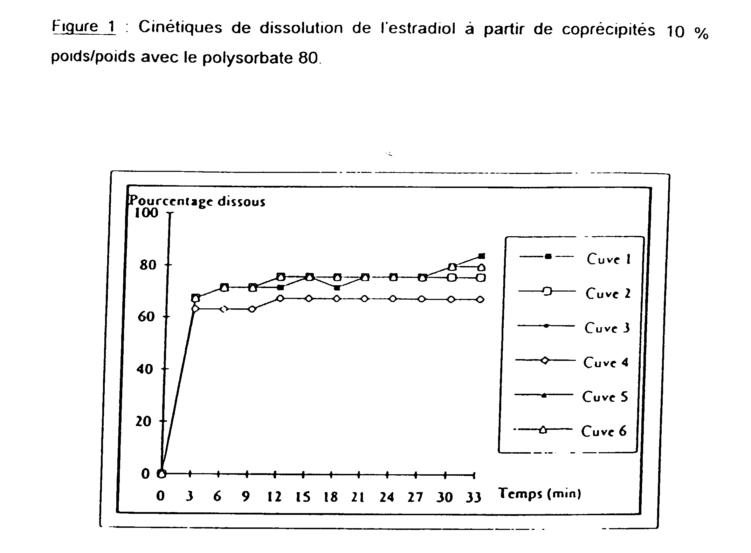

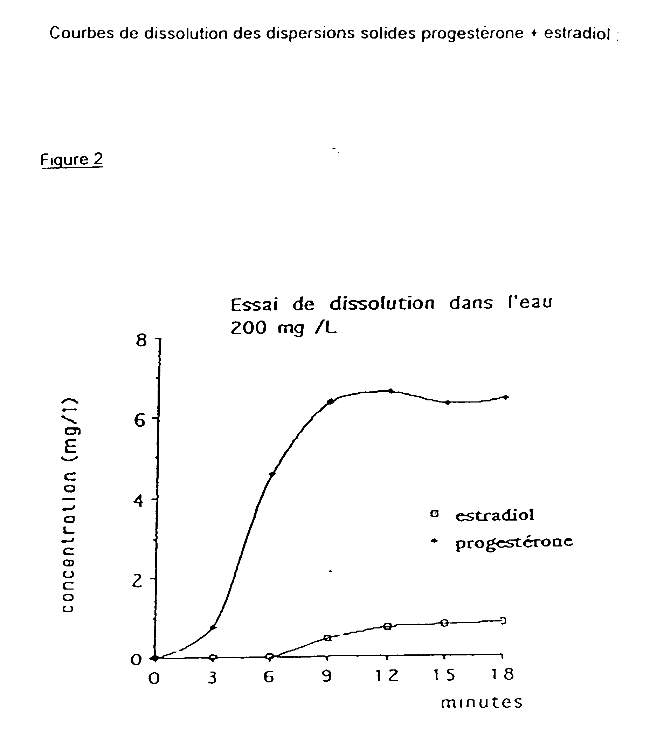

Les cinétiques de dissolution de l'estradiol à partir des dispersions solides à 10% poids/poids sont représentées figure 1.

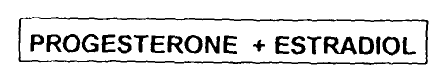

### I - PREPARATION DES DISPERSIONS SOLIDES SELON L'INVENTION

### A- Matières premières

Les matières premières employées sont les mêmes que que celles utilisées dans le cas de l'estardiol ajoutées à la progetérone (USP EP, lot 347 JA), UPJOHN Company, Fine chemical division, Kalamazoo, Michigan 49001 USA.

### B- Le matériel

Le matériel est égalemement le même que celui utilisé dans le cas de l'estradiol.

### C- Préparation des dispersions solides par dissolution-évaporation

Les coprécipités réalisés ont la composition suivante :
2,5 g de progestérone
0,05 g d'estradiol
2,2 g de PVP
0,25 g soit 50 ml d'une solution à 5g/l de Tween 80 dans de l'alcool absolu
103 ml d'alcool absolu nécessaire pour la dissolution totale des différents composés (50 ml d'alcool absolu pour 1 g de progestérone, 10 ml pour 1 g d'estradiol).

Les dispersions solides sont obtenues par évaporation du solvant sous vide (P = 200 millibars) durant 25 minutes puis la pression est diminuée à son minimum (environ 40 millibars) pendant 35 minutes. La température du bain-marie est de 60°C et la vitesse de rotation du ballon est de 100 tours / minute.

Les échantillons ainsi récupérés sont placés dans un dessicateur pendant 48 heures puis sont broyés et tamisés. Les essais galéniques sont effectués sur des échantillons de granulométrie inférieure à 100 micromètres.

Les coprécipités obtenus avant le passage du dessicateur, sont sous forme de bloc cristallin, jaune pâle ou blanc et l'on récupère également des petits « amas » cristallisés

### II - ETUDE DES CINETIQUES DE DISSOLUTION

Ces essais de dissolution ont été réalisés dans l'eau.
Les coprécipités utilisés contiennent 50% de progestérone et 1% d'estradiol. Deux essais ont été réalisés : l'un à 200 mg de poudre / litre d'eau et l'autre à 20 mg de poudre par litre d'eau. La réalisation de ces deux essais s'expliquent par le fait que l'estradiol n'est pas détectable pour une prise d'essai de 20 mg de poudre / litre d'eau et qu'il a donc été nécessaire de réaliser une prise d'essai à 200 mg de poudre / litre d'eau afin de pouvoir détecter l'estradiol.

### RESULTATS

### 1. Essai 1 (200 mg de poudre / litre d'eau)

| Temps (min) | Teneur en estradiol | | | Teneur en progestérone | | |
|---|---|---|---|---|---|---|
| | mg/l | Pourcentage par rapport à la teneur théorique | Pourcentage par rapport à la teneur mesurée | mg/l | Pourcentage par rapport à la teneur théorique | Pourcentage par rapport à la teneur mesurée |
| 3 | < 0,3 | < 15 | < 25 | 0,78 | 0,8 | 1,0 |
| 6 | < 0,3 | < 15 | < 25 | 4,56 | 4,6 | 6,0 |
| 9 | 0,46 | 23 | 38,3 | 6,33 | 6,3 | 8,3 |
| 12 | 0,76 | 38 | 63,3 | 6,58 | 6,6 | 8,6 |
| 15 | 0,82 | 41 | 68,3 | 6,30 | 6,3 | 8,2 |
| 18 | 0,86 | 43 | 71,7 | 6,43 | 6,4 | 8,4 |

### 2. Essai 2 (20 mg de poudre / litre d'eau)

| Temps (min) | Teneur en estradiol | | | Teneur en progestérone | | |
|---|---|---|---|---|---|---|
| | mg/l | Pourcentage par rapport à la teneur théorique | Pourcentage par rapport à la teneur mesurée | mg/l | Pourcentage par rapport à la teneur théorique | Pourcentage par rapport à la teneur mesurée |
| 3 | ND | - | - | 0,27 | 2,7 | 3,5 |
| 6 | ND | - | - | 2,99 | 29,9 | 39,0 |
| 9 | ND | - | - | 3,87 | 38,7 | 50,5 |
| 12 | ND | - | - | 4,28 | 42,8 | 55,9 |
| 15 | ND | - | - | 4,49 | 44,9 | 58,6 |
| 18 | ND | - | - | 4,54 | 45,4 | 59,3 |
| ND : Non détectable | | | | | | |

### 3. Profils de dissolution

Les courbes de dissolution en fonction du temps sont présentées ci-après.

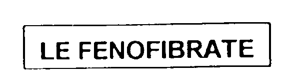

### I- PREPARATION DES DISPERSIONS SOLIDES SELON L'INVENTION

### A- Matières premières

- le fénofibrate (lot F009ZX100)
   (Schweizerhall France Deshors, 57 bd de Montmorency, 75016 Paris).
- la polyvinylpirrolidone (Kollidon 30, Nr 56.0902)
   (BASF Aktiengesellschaft, 67056 Ludwigshafen, Germany).
- Tween 80 (polyoxyethylene sorbitan monooleate): lot AB 397
   (ICI Chemicals, Niederlassuny der Deutsche ICI Gmbh, Goldschmidtstr 100 D 4300 Essen 1).
- l'alcool absolu (Distillerie HAUGUEL, 76700 Gonfreville l'Orcher).

### B- Matériel

Le matériel est exactement le même que celui qui a servi à faire les coprécipités précédents.

### C- Préparation des dispersions solides par dissolution-évaporation

Cinq séries de coprécipités sont réalisées, chaque série comporatnt 6 échantillons.

| *Séries* | *1* | *2* | *3* | *4* | *5* |
|---|---|---|---|---|---|
| *Fénofibrate* | 2.5 gr | 2 gr | 1.5 gr | 1 gr | 0.5 gr |
| *PVP K30* | 2.25 gr | 2.75 gr | 3.25 gr | 3.75 gr | 4.25 gr |
| *Tween 80* | 0.25 gr | 0.25 gr | 0.25 gr | 0.25 gr | 0.25 gr |
| *Alcool absolu* | 103 ml | 81 ml | 61 ml | 41 ml | 21 ml |

Les dispersions solides sont obtenues par évaporation sous vide (P=205 millibars) durant 24 minutes puis la pression est diminuée à son minimum (40 millibars) pendant 25 minutes. La température du bain marie est de 60°C et la vitesse de rotation du ballon est de 100 tours/minute.

Les temps d'évaporation sont adaptés suivant la quantité d'alcool absolu ajoutée pour dissoudre les produits, donc suivant la série de coprécipités.

Les échantillons à 2,5 g de fénofibrate ont un aspect jaunâtre et forment une boule plus ou moins visqueuse après une heure d'évaporation à pression minimum.
Plus la teneur en fénofibrate diminue (la quantité d'alcool absolu ajoutée diminuant de par ce fait), plus les échantillons récupérées sont friables, sous forme de poudre fine ou de granules. La couleur est blanche et brillante et les coprécipités semblent très secs.
Les échantillons ainsi récupérés sont placés dans un dessicateur pendant 48 heures puis sont broyés et tamisés. Les essais galéniques sont effectués sur des échantillons de granulométrie inférieure à 100 micromètres.

### II - ETUDE DES CINETIQUES DE DISSOLUTION

### A- Technique expérimentale

Celle-ci est identique à celle utilisée pour l'étude de la cinétique de dissolution des coprécipités d'estradiol.

### B- Résultats

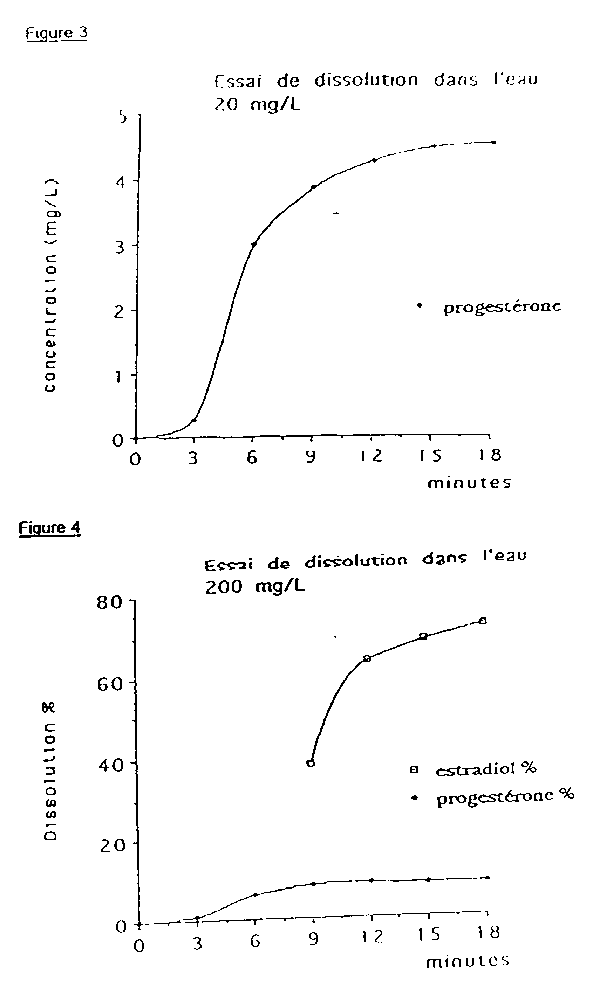

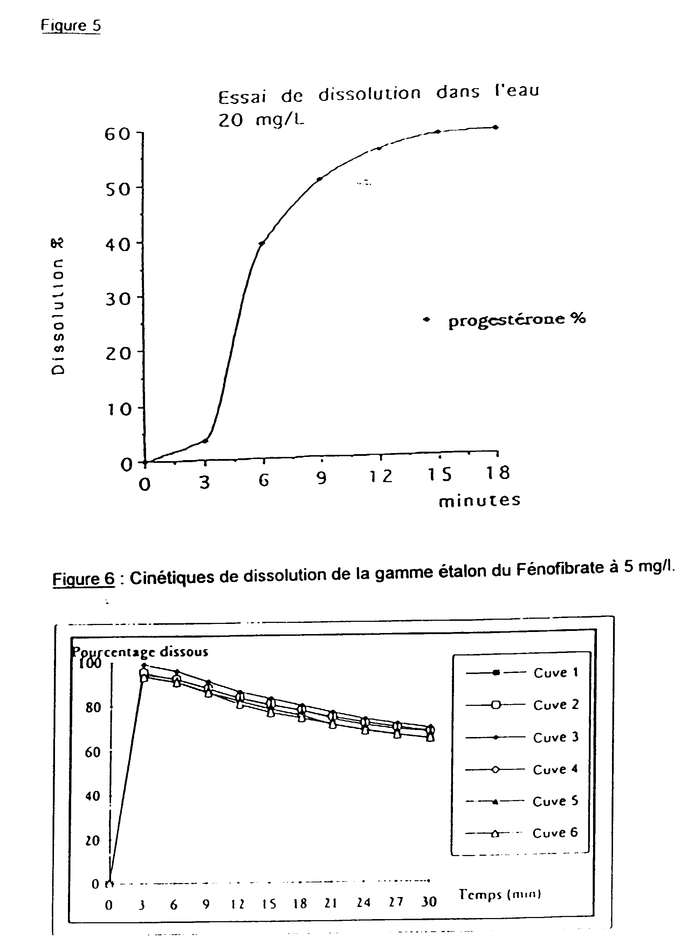

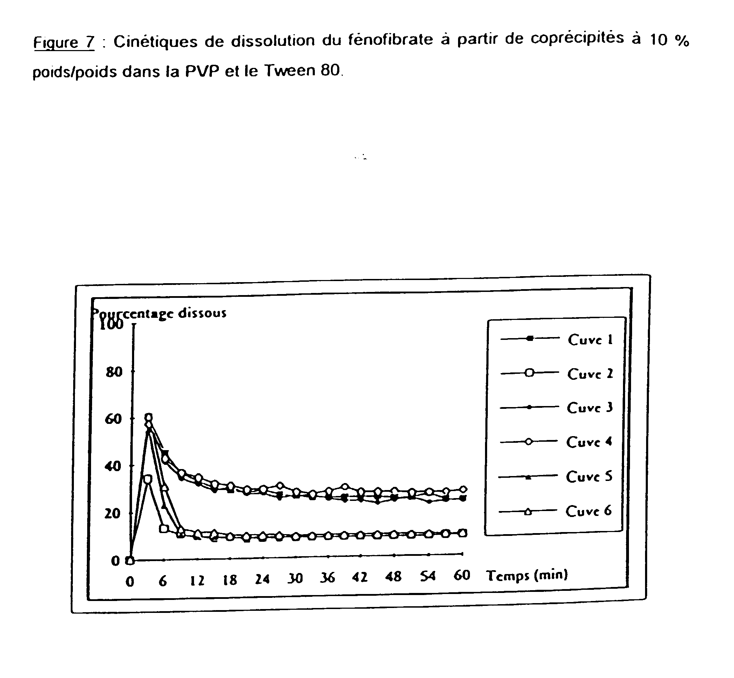

Nous avons mesuré précédemment la solubilité maximale du fenofibrate dans l'eau. Celle-ci est inférieure à 3 mg/l.
Les coprécipités fabriqués ci-dessus sont successivement à 50 %, 40 %, 30 %, 20 % et 10 % en poids de fenofibrate. Si l'on veut introduire dans les éracteurs une quantité de fenofibrate permettant sa solubilisation totale dans 1 litre d'eau, il faudrait pouvoir peser respectivement 6 mg, 7,5 mg, 10 mg, 15 mg, et 30 mg de coprécipités.
Cette précision dans la mesure liée aux pertes infimes mais inévitables par manipulation ne peut être obtenue.
C'est pourquoi nous choisissons de faire une gamme étalon à 5 mg/l comme pour la progestérone et effectuons les cinétiques de dissolution en introduisant une quantité équivalente en coprécipités. Cette étude va nous permettre de quantifier la qualité du fénofibrate solubilisée et nous observerons une recristallisation du fénofibrate au cours de l'essai.
Sont représentées figure 3 et 4 respectivement , la gamme étalon et la cinétique de dissolution du fénofibrate dans les coprécipités à 10 % poids/poids (série choisie au hasard et représentataive d'un phénomène commun aux autres coprécipités).

| Temps (minutes) | Gamme étalon Fénofibrate à 5 mg/l | | | | | |
|---|---|---|---|---|---|---|
| | Cuve 1 | Cuve 2 | Cuve 3 | Cuve 4 | Cuve 5 | Cuve 6 |
| 0 | 0 | 0 | 0 | 0 | 0 | 0 |
| 3 | 95.26 | 95.26 | 98.94 | 93.68 | 94.21 | 93.15 |
| 6 | 92.1 | 92.1 | 95.78 | 91.05 | 92.1 | 90.52 |
| 9 | 87.89 | 87.89 | 91.05 | 86.31 | 87.89 | 85.78 |
| 12 | 83.68 | 83.68 | 86.31 | 82.1 | 83.15 | 80.52 |
| 15 | 80.52 | 80.52 | 83.15 | 78.42 | 80 | 76.84 |
| 18 | 77.89 | 77.36 | 80 | 75.26 | 77.36 | 74.21 |
| 21 | 74.73 | 74.73 | 76.84 | 71.57 | 73.68 | 71.05 |
| 24 | 72.1 | 72.1 | 73.68 | 68.94 | 71.05 | 68.42 |
| 27 | 70 | 70 | 71.57 | 66.84 | 68.94 | 66.31 |
| 30 | 68.42 | 67.89 | 69.47 | 64.73 | 67.36 | 64.73 |

La décroissance de cette cinétique de dissolution est exclusivement due à la sensibilité de la formulation à la lumière et au fait que le dosage est réalisé par spectrophotométrie UV à 278 nm, ce qui entraine une dégradation de la formulation. Cette dégradation n'est donc absolument pas due à la technique de coprécipité mais à la technique de lecture.

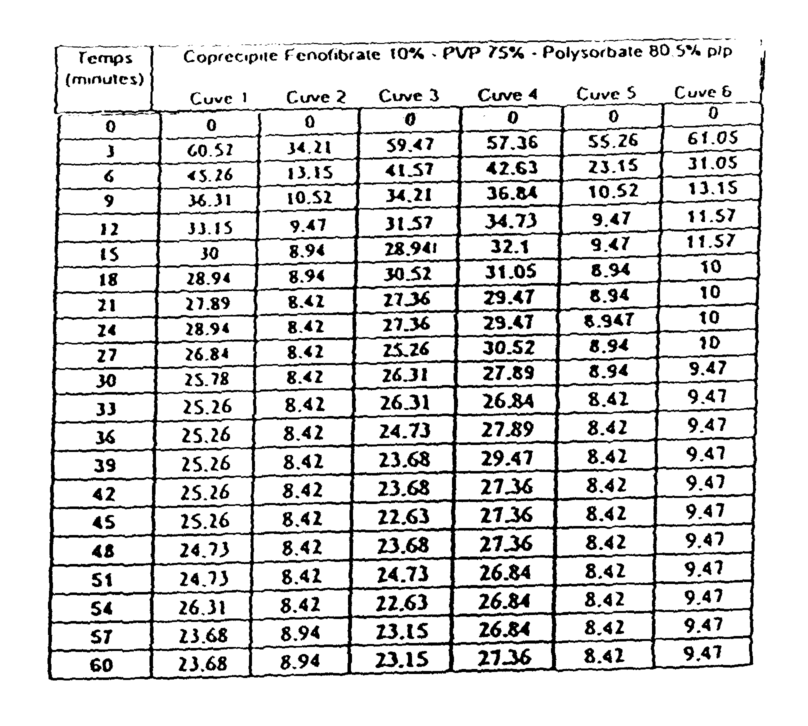

Ici aussi, la spectrophotométrie UV est entièrement responsable de la décroissance de la cinétique de dissolution.

### V - DISPERSIONS SOLIDES CONTENANT DE L'ACETATE DE MEDROXYPROGESTERONE

### 1 - Production des dispersions solides selon l'invention

Cinq séries de co-précipités ont été réalisées chacune étant formée de six échantillons.

La quantité d'acétate de médroxyprogestérone dans chacune d'entre elles était de 0,5 g et celle de polyvinylpyrrolidone s'échelonnait de 2 à 5 g. Les dispersions solides ont été préparées selon le mode opératoire décrit à l'exemple IV. La solubilité de l'acétate de médroxyprogestérone dans l'eau est très faible. Le dosage de la concentration en acétate de médroxyprogestérone ainsi dissoute dans l'eau a été effectué par spectrophotométrie en UV à 241 nm.

Le pourcentage de composé dissout a été à peu près total en 30 minutes à température ordinaire.
**1. BLOCH D.W - SPEISER P.P**
   Solid dispersions. Fundamentals and examples
   Pharm. Acta Helv. 62, 1, 1, 23-27 (1987)
**2. CHIOU C.I - RIEGELMAN S.**
   Pharmaceutical applications of solid dispersion systems
   J. Pharm. Sci. 60, 9, 1281-1302 (1971)
**3. DUCHENE D.**
   Les dispersions solides. Stabilité et conservation
   STP Pharma. 1. (11) 1064-1073 (1985)
**4. DUCHENE D.**
   Les dispersions solides : intérêt et limites
   Les entretiens du Carla, Tome VII, 99-107 (1986)
**5. DUCLOS R.**
   Amélioration de la biodisponibilité de la progestérone naturelle par utilisation de dispersions solides dans le polyoxyéthylène glycol 6000. Thèse de doctorat d'Etat es Sciences Pharmaceutiques, Université de ROUEN (1989)
**6. FORD J.L**
   The current status of solid dispersions
   Pharm. Acta. Helv. 6, 3, 69-88 (1986)
**7. PUISIEUX F - HENRY S.**
   Les dispersions solides
   Labo. Pharma Prob. et Tech. 305, 11-20 (1981)
**8. SEKIGUCHI K. - OBI N.**
   Studies on absorption of eutectic mixtures. A comparison of the behavior of eutectic mixture of sulfathiazole and that of ordinary sulfathiazole in man. Chem. Pharm. Bull. 9, 866-872 (1961)

## Revendications

1. Procédé d'obtention de dispersions solides d'agents médicamenteux dans un agent transporteur hydrophile assurant une meilleure solubilité dans les milieux aqueux et une meilleure résorption dans le tube digestif caractérisé en ce que l'on dissout le principe actif médicamenteux dans un solvant organique volatil contenant un amide cyclique très hydrophile puis en ce que la solution organique ainsi réalisée est évaporée à sec, le coprécipité obtenu est broyé, tamisé, dilué avec un excipient ou un véhicule pharmaceutiquement-acceptable, ou bien utilisé tel quel.

2. Procédé selon la revendication 1° dans lequel l'évaporation à sec de la solution organique est effectuée par lit d'air fluidisé.

3. Procédé selon la revendication 1° ou la revendication 2° dans lequel le solvant organique volatil est un solvant oxygéné ou un solvant chloré, ou un mélange de plusieurs solvants.

4. Procédé selon l'une des revendications 1 à 3° dans lequel le solvant organique volatil est un alcanol.

5. Procédé selon l'une des revendications 1 à 3° dans lequel le solvant organique volatil est le chlorure de méthylène.

6. Procédé selon l'une des revendications 1 à 4° dans lequel le solvant volatil est l'éthanol, l'isopropanol ou le terbutanol.

7. Procédé selon l'une des revendications 1 à 6° dans lequel l'amide cyclique très hydrophile est choisi dans le groupe constitué par les polyvinylpyrrolidones, la N-méthylpyrrolidone, le N-méthyl caprolactame et la N-méthyl pipéridinone-2.

8. Procédé selon l'une des revendications 1 à 6° dans lequel les polyvinylpyrrolidones sont de préférence celles qui ont un poids moléculaire de l'ordre de 10 000 à 50 000.

9. Procédé selon l'une des revendications 1 à 8° dans lequel le mélange solvant est additionné d'un agent tensioactif.

10. Procédé selon la revendication 9° dans lequel l'agent tensioactif est un agent tensioactif non ionique.

11. Procédé selon la revendication 9° ou la revendication 10° dans lequel l'agent tensioactif non ionique est choisi parmi les esters polyoxyéthyléniques de sorbitanne et d'acides gras, saturés ou non saturés, ayant au moins 8 atomes de carbone, les éthers polyoxyéthyléniques d'alcool gras ayant au moins 8 atomes de carbone et, les esters polyoxyéthyléniques d'acide stéarique.

12. Procédé selon l'une des revendications 9° à 11° dans lequel l'agent tensioactif est choisi dans le groupe constitué par les polysorbates et les cremophors.

13. Procédé selon l'une des revendications 1° à 12° dans lequel l'agent tensioactif est présent à une concentration variant de 0,5 à 20 % en poids.

14. Une dispersion solide obenue selon l'une des revendications 1 à 13° dans laquelle le principe actif est choisi parmi la progestérone, l'estradiol, l'association progestérone-estradiol, le fénofibrate, le métronidazole, la pipéracilline, le cefuroxime, la cyclosporine et l'itraconazole.

15. Une dispersion solide selon la revendication 14° dans laquelle le principe actif est la progestérone.

16. Une dispersion solide selon les revendications 14° et 15° dans laquelle le principe actif est la progestérone à une concentration variant de 20 à 60% en poids/poids.

17. Une dispersion solide selon la revendication 14° dans laquelle le principe actif est l'estradiol.

18. Une dispersion solide selon les revendications 14° et 17° dans laquelle le principe actif est l'estradiol à une concentration variant de 5 à 20% en poids/poids.

19. Une dispersion solide selon la revendication 14° dans laquelle le principe actif est un mélange quelconque de progestérone et d'estradiol.

20. Une dispersion solide selon les revendications 14° et 19° dans laquelle le principe actif est un mélange quelconque de progestérone et d'estradiol à une concentration variant de 20 à 60% en poids/poids pour la progestérone et de 5 à 20% en poids/poids pour l'estradiol.

21. Une dispersion solide selon la revendication 14° dans laquelle le principe actif est le fénofibrate.

22. Une dispersion solide selon les revendications 14° et 21° dans laquelle le principe actif est le fénofibrate à une concentration variant de 10 à 50% en poids/poids.

23. Une dispersion solide selon la revendication 14, dans laquelle le principe actif est l'acétate de medroxyprogestérone.

24. Une dispersion solide selon les revendications 14° à 22° dans laquelle l'agent tensioactif est présent à une concentration variant de 0,5 à 20% en poids.

25. Compositions pharmaceutiques renfermant les dispersions solides selon l'une des revendications 14° à 23° caractérisées en ce que la dispersion solide de principe actif est mélangée à un excipient ou un diluant inerte, non toxique pharmaceutiquement-acceptable.

26. Une composition pharmaceutique selon la revendication 24°, caractérisée en ce que la dispersion de coprécipité est pulvérisée sur des microgranules neutres qui sont ensuite pelliculés à l'aide d'un dérivé de cellulose.
